# EUROPEAN PATENT APPLICATION

(11) **EP 3 093 299 A1**
(43) Date of publication of application: **16.11.2016**
(21) Application number: 15733094.5
(22) Date of filing: 06.01.2015
(51) Int. Cl.: C08F 2/01, C08F 20/10, C08J 3/075, C08J 3/24

(54) **METHOD FOR PREPARING SUPERABSORBENT POLYMER**

(30) Priority: 06.01.2014 KR 20140001256; 24.12.2014 KR 20140188849
(71) Applicant: Hanwha Chemical Corporation, Seoul 100-797 (KR)
(72) Inventor: SIM, Yu Jin, Daejeon 305-720 (KR); KIM, Eui Duk, Daejeon 305-720 (KR); KIM, Ji Yeon, Dalseong-gun Daegu 711-765 (KR); PAIK, Choong Hoon, Daejeon 305-720 (KR); OH, Seok Heon, Daejeon 305-720 (KR); LEE, Min Ho, Pohang-si Gyeongsangbuk-do 791-786 (KR); CHOI, Dae Keon, Jeonju-si Jeollabuk-do 560-792 (KR)
(74) Representative: Glawe, Delfs, Moll
(86) International application number: PCT/KR2015/000080
(87) International publication number: WO 2015/102457

(57) **Abstract**

The present invention relates to a method for preparing a superabsorbent polymer. The method for preparing a superabsorbent polymer according to the present invention comprises the steps of: polymerizing monomer compositions in at least two polymerization reactors; grinding the polymers obtained in the polymerization step; drying the polymers; and mixing the polymers.

## Description

### [Technical Field]

The present invention relates to a method for preparing a superabsorbent polymer.

### [Background Art]

A superabsorbent polymer (SAP) is a synthetic polymer material capable of absorbing approximately 500 to 1000 times its weight of water. The superabsorbent polymer has been differently called a superabsorbency material (SAM), an absorbent gel material (AGM), etc. by different development enterprises. The superabsorbent polymer disclosed above started to be commercialized for sanitary items, and is now being used widely as a water combination soil for horticulture, a water-stop material for civil engineering and construction, a nursery sheet, a freshness preservative in the food distribution field, a poultice material, and the like in addition to the sanitary fittings like a paper diaper for a child.
An inverse suspension polymerization method or an aqueous polymerization method is known as a method for preparing the above superabsorbent polymer. For example, inverse suspension polymerization is disclosed in Japanese Patent Publication Nos. sho 56-161408, sho 57-158209, and sho 57-198714. As the aqueous polymerization method, a thermal polymerization method of polymerizing an aqueous solution by applying heat to the aqueous solution and a photopolymerization method of polymerizing an aqueous solution by irradiating ultraviolet (UV) light to the aqueous solution are known.

### [Disclosure]

### [Technical Problem]

Aspects of the present invention provide a superabsorbent polymer with excellent physical properties.

However, aspects of the present invention are not restricted to the one set forth herein. The above and other aspects of the present invention will become more apparent to one of ordinary skill in the art to which the present invention pertains by referencing the detailed description of the present invention given below.

### [Technical Solution]

According to an exemplary embodiment of the invention to solve the technical problem, a method for preparing a superabsorbent polymer, the method comprising the steps of: polymerizing monomer compositions in at least two polymerization reactors; grinding the polymers obtained in the polymerization step; drying the polymers; and mixing the polymers.

The step of mixing the polymers may be performed after the step of polymerizing the monomer compositions.

The step of polymerizing the monomer compositions may be performed using belt reactors or kneader reactors.

The method may further comprise the step of cutting the polymers before the step of grinding the polymers.

The monomer compositions respectively in the at least two polymerization reactors may be different in composition.

The monomer compositions respectively in the at least two polymerization reactors may be different in type of crosslinking agents, content of crosslinking agents, or type and content of crosslinking agents.

The at least two polymerization reactors are continuous polymerization reactors.

The method may further comprise the step of crosslinking a surface of a superabsorbent polymer.

Specific details of other embodiments are included in the detailed descriptions and drawings.

### [Advantageous Effects]

Embodiments of the present invention provide at least one of the following advantages.

A preparation method according to the present invention can provide a superabsorbent polymer with excellent physical properties and improve productivity.

However, the effects of the present invention are not restricted to the one set forth herein. The above and other effects of the present invention will become more apparent to one of daily skill in the art to which the present invention pertains by referencing the claims.

### [Best Mode]

Advantages and features of the present invention and methods of accomplishing the same may be understood more readily by reference to the following detailed description of exemplary embodiments and the accompanying drawings. The present invention may, however, be embodied in many different forms and should not be construed as being limited to the embodiments set forth herein. Rather, these embodiments are provided so that this disclosure will be thorough and complete and will fully convey the concept of the invention to those skilled in the art, and the present invention will only be defined by the appended claims. Like reference numerals refer to like elements throughout the specification. In the drawings, sizes and relative sizes of layers and regions may be exaggerated for clarity.

It will be understood that, although the terms first, second, third, etc., may be used herein to describe various elements, these elements should not be limited by these terms. These terms are only used to distinguish one element from another element. Thus, a first element discussed below could be termed a second element without departing from the teachings of the present invention.

### Method for preparing a superabsorbent polymer

A method for preparing a superabsorbent polymer according to an embodiment of the present invention includes the steps of polymerizing in at least two polymerization reactors, grinding the polymers obtained in the polymerization step, drying the polymers, and mixing the polymers.

The step of polymerizing a superabsorbent polymer is not particularly limited, but may be polymerizing by injecting a monomer compositions into polymerizers. For efficient processes, the monomer compositions may be polymerized in a continuous manner using continuous polymerization reactors. In this case, to form a superabsorbent polymer, the monomer compositions may be injected and polymerized on belts or may be polymerized using kneader reactors. However, the present invention is not limited thereto.

Any monomer usually used to prepare a superabsorbent polymer may be used as a water-soluble ethylene-based unsaturated monomer contained in a monomer composition. The monomer may be at least one selected from the group consisting of an anionic monomer and a salt thereof, a nonionic hydrophilic monomer, and an amino group-containing unsaturated monomer and a quaternary compound thereof.

In an exemplary embodiment, the monomer may be at least one anionic monomer, which is selected from the group consisting of acrylic acid, methacrylic acid, maleic anhydride, fumaric acid, crotonic acid, itaconic acid, 2-acryloylethane sulfonic acid, 2-methacryloylethane sulfonic acid, 2- (meth)acrylo-ylpropane sulfonic acid and 2-(meth)acrylamide-2-methyl propane sulfonic acid, or a salt thereof; at least one nonionic hydrophilic monomer which is selected from the group consisting of (meth)acrylamide, N-substituted (meth)acrylate, 2-hydroxyethyl(meth)acrylate, 2- hydroxypropyl(meth)acrylate, methoxy polyethylene glycol (meth)acrylate and polyethylene glycol (meth)acrylate; or at least one amino group-containing unsaturated monomer, which is selected from the group consisting of (N,N)-dimethylaminoethyl(meth)acrylate and (N,N)- dimethylaminopropyl(meth)acrylamide, or a quaternary compound thereof.

The concentration of the water-soluble ethylene-based unsaturated monomer in the monomer composition may be determined in view of polymerization time and reaction conditions (such as the feeding speed of the monomer composition, the irradiation time, range and intensity of heat and/or light, and the width, length and movement speed of a belt). In an exemplary embodiment, the concentration of the water-soluble ethylene-based unsaturated monomer may be in a range of 40 to 60% by weight. In this case, it may be efficient in terms of monomer solubility and economic feasibility.

The monomer composition may further include at least one additive selected from the group consisting of a photopolymerization initiator, a thermal polymerization initiator, and a crosslinking agent. The type of the polymerization initiator may be determined based on whether thermal polymerization, photopolymerization, or both thermal polymerization and photopolymerization will be used.

The photopolymerization initiator is not particularly limited but may be a single material or a mixture of two or more materials selected from the group consisting of, but is not limited to, an acetophenone derivative such as diethoxy acetophenone, 2-hydroxy-2-methyl-1-phenylpropane-1-on, 4-(2-hydroxy-ethoxy)phenyl-(2-hydroxy)-2-propyl ketone or 1-hydroxycyclohexyl phenyl ketone; a benzoin alkyl ether compound such as benzoin methyl ether, benzoin ethyl ether, benzoin isopropyl ether or benzoin isobutyl ether; a benzophenone derivative such as o-benzoyl methyl benzoate, 4-phenyl benzophenone, 4-benzoyl-4'-methyl-diphenyl sulfide or (4-benzoyl benzyl)trimethyl ammonium chloride; a thioxanthone compound; an acyl phosphine oxide derivative such as bis(2,4,6-trimethylbenzoyl)-phenyl phosphine oxide, diphenyl(2,4,6-trimethylbenzoyl)-phosphine oxide or diphenyl(2,4,5-trimethylbenzoyl)-phosphine oxide; and an azo compound such as 2-hydroxy methyl propionitrile or 2,2'-(azobis(2-methyl-N-(1,1-bis(hydroxymethyl)-2-hydroxyethyl)propionamide).

The thermal polymerization initiator is not particularly limited but may be a single initiator or a mixture of two or more initiators selected from the group consisting of, but not limited to, an azo-based initiator, a peroxide-based initiator, a redox-based initiator, and an organic halide. The thermal polymerization initiator may also be, but is not limited to, sodium persulfate (Na₂S₂O₈) or potassium persulfate (K₂S₂O₈).

Each of the photopolymerization initiator and the thermal polymerization initiator can be added in the monomer composition in any amount as long as it can bring about a polymerization initiating effect. In an exemplary embodiment, the photopolymerization initiator may be added in an amount of, but not limited to, 0.005 to 0.1 parts by weight based on 100 parts by weight of monomer, and the thermal polymerization initiator may be added in an amount of, but not limited to, 0.01 to 0.5 parts by weight based on 100 parts by weight of monomer.

The crosslinking agent may be a crosslinking agent containing at least one functional group that can react with a substituent of a monomer and at least one ethylene unsaturated group or a crosslinking agent containing two or more functional groups that can react with a substituent of a monomer and/or a substituent formed by hydrolyzing the monomer.

In an exemplary embodiment, the crosslinking agent may be bisacrylamide having a carbon number of 8 to 12, bismethacrylamide having a carbon number of 8 to 12, poly(meth)acrylate of polyol having a carbon number of 2 to 10, or poly(meth)allyl ether of polyol having a carbon number of 2 to 10. More specifically, the crosslinking agent may be a single material or a mixture of two or more materials selected from the group consisting of, but not limited to, N,N'-methylenebis (meth)acrylate, ethylene oxy(meth)acrylate, polyethylene oxy(meth)acrylate, propylene oxy (meth)acrylate, glycerin diacrylate, glycerin triacrylate, trimethylol triacrylate, tri-allylamine, triallyl cyanurate, triallyl isocyanate, polyethylene glycol, diethylene glycol, propylene glycol, polyethylene glycol diacrylate, and trimethylolpropane triacrylate.

The crosslinking agent can be added in the monomer composition in any amount as long as it can bring about a crosslinking effect. In an exemplary embodiment, the crosslinking agent may be added in an amount of, but not limited to, 0.01 to 0.5 parts by weight based on 100 parts by weight of monomer.

The monomer compositions respectively injected into the at least two polymerization reactors may be different in composition. In an exemplary embodiment, the monomer compositions respectively in the at least two polymerization reactors may be different in type of crosslinking agents, content of crosslinking agents, or type and content of crosslinking agents.

The polymerized superabsorbent polymer may be injected into a cutting apparatus and then cutting by a cutter.

The cut superabsorbent polymer may be ground and dried, and then the dried polymer may be ground again. In some cases, a pre-drying process may be performed before the grinding process in order to prevent clumping in the grinding process.

A grinding method used here is not limited to a particular method. For example, a device for cutting and extruding a rubber elastomer may be used. In an exemplary embodiment, the grinding method may be, but is not limited to, a typical cutter, a chopper-type cutter, a kneader-type cutter, a vibration grinder, an impact grinder, or a friction grinder.

As a drying method, a conventional dryer and a heating furnace may be used. In an exemplary embodiment, the drying method may be, but is not limited to, a hot-air dryer, a fluidized bed dryer, a flash dryer, a UV dryer, or a dielectric-heat dryer. A drying temperature is not particularly limited but may be in a range of 100 to 200 °C in order for prevention of thermal degradation and efficient drying. In addition, a drying time may be in a range of, but not limited to, 40 to 80 minutes.

In an exemplary embodiment, the method for preparing a superabsorbent polymer may further include the step of crosslinking a surface of the superabsorbent polymer.

The step of crosslinking the surface of the superabsorbent polymer may be performed using, but not limited to, ethylene glycol diglycidyl ether, water, and ethanol.

The step of crosslinking the surface of the superabsorbent polymer may be performed after the formation of particles through, e.g., grinding and drying. However, the present invention is not limited thereto, and the step of crosslinking the surface of the superabsorbent polymer can be performed a number of times if necessary.

The step of mixing the polymers can be performed at any stage after the polymerization step. That is, the step of mixing the polymers can be performed immediately after the polymerization step, immediately after the cutting step, immediately after the grinding step, immediately after the drying step, or immediately after the surface-crosslinking step.

### [Mode for Invention]

### Comparative Example 1

84 g of caustic soda (NaOH) 50 % and 98.3 g of water were put into a reaction vessel and stirred and diluted. Then, 107.7 g of acrylic acid was added to neutralize the mixture of caustic soda and water. After the temperature increased by heat of neutralization was lowered to 40 °C, 0.1 g of polyethylene glycol diacrylate which is an internal crosslinking agent and 0.36 g of diphenyl (2,4,5-trimethylbenzoyl)- phosphine oxide 3 % solution which is a UV initiator were added and mixed. In addition, 1.08 g of potassium persulfate 3 % solution which is a thermal initiator was added. After that, polymerization was carried out for three minutes using a UV lamp with an intensity of 8 mW/cm². The resultant polymerized polymer in a gel state was cut using a chopper and dried for one hour in a 180 °C hot-air oven. The polymer which became hard by drying was ground using a grinder and then sorted according to a size of 150 to 850 µm, thereby producing a superabsorbent polymer.

The superabsorbent polymer was surface-crosslinked using ethylene glycol diglycidyl ether, water, and ethanol. Then, the superabsorbent polymer was made to react for 40 minutes at a temperature of 140 °C and then ground. A surface-treated superabsorbent polymer with a grain size of 150 to 850 µm was produced using a sieve.

### Comparative Example 2

A superabsorbent polymer was prepared in the same way as in Comparative Example 1 except that 0.1 g of trimethylolpropane triacrylate was used as a crosslinking agent.

### Preparation Example 1

The composition of Comparative Example 1 was prepared in one vessel, and the composition of Comparative Example 2 was prepared in another vessel. Then, the compositions were polymerized on separate conveyor belts. After the polymerized compositions were cut separately, they were mixed in a hot-air belt dryer. Finally, one surface-crosslinking process was performed to produce a surface-treated superabsorbent polymer.

### Experimental Example 1

The centrifuge retention capacity (CRC), absorbency under pressure (AUP) and extractable content (EC) of a superabsorbent polymer prepared in each of Preparation Example 1, Comparative Example 1 and Comparative Example 2 were measured according to EDANA WSP 241.2.R3, EDANA WSP 242.2.R3 and EDANA WSP 270.2.R3 standards. The measurement results are shown in Table 1 below.

**[Table 1]**

| | CRC | AUP (0.7 psi) | EC (1h) |
|---|---|---|---|
| Comparative Example 1 | 42 | 17 | 17.2 |
| Comparative Example 2 | 29 | 27 | 2.2 |
| Preparation Example 1 | 35 | 24 | 5.6 |

Generally, an excellent CRC may lead to a high absorption capacity, and a gel blocking phenomenon may not occur in the case of a high AUP and a low EC.

Comparative Example 1 has a high absorption capacity due to a high CRC. However, the low AUP and high EC of Comparative Example 1 can reduce an absorption capacity under pressure and cause the gel blocking phenomenon. Comparative Example 2 may not have the gel blocking phenomenon due to its high AUP and low EC. However, due to a low CRC, Comparative Example 2 has too low an absorption capacity to be used alone. Preparation Example 1 has a superior absorption capacity and a not low absorption capacity under pressure. In addition, there is no concern about the gel blocking phenomenon because of a relatively low EC.

While the present invention has been particularly shown and described with reference to exemplary embodiments thereof, it will be understood by those of ordinary skill in the art that various changes in form and detail may be made therein without departing from the spirit and scope of the present invention as defined by the following claims. The exemplary embodiments should be considered in a descriptive sense only and not for purposes of limitation.

## Claims

1. A method for preparing a superabsorbent polymer, the method comprising the steps of:
polymerizing monomer compositions in at least two polymerization reactors;
grinding the polymers obtained in the polymerization step;
drying the polymers; and
mixing the polymers.

2. The method of claim 1, wherein the step of mixing the polymers is performed after the step of polymerizing the monomer compositions.

3. The method of claim 1, wherein the step of polymerizing the monomer compositions is performed using belt reactors or kneader reactors.

4. The method of claim 1, further comprising the step of cutting the polymers before the step of grinding the polymers.

5. The method of claim 1, wherein the monomer compositions respectively in the at least two polymerization reactors are different in composition.

6. The method of claim 5, wherein the monomer compositions respectively in the at least two polymerization reactors are different in type of crosslinking agents, content of crosslinking agents, or type and content of crosslinking agents.

7. The method of claim 1, wherein the at least two polymerization reactors are continuous polymerization reactors.

8. The method of claim 1, further comprising the step of crosslinking a surface of a superabsorbent polymer.
